# EUROPEAN PATENT SPECIFICATION

(11) **EP 0 967 988 B1**
(45) Date of publication and mention of the grant of the patent: **03.04.2002**
(21) Application number: 98904453.2
(22) Date of filing: 25.02.1998
(51) Int. Cl.: A61K 35/78

(54) **OINTMENT FOR THE TREATMENT OF BURNS AND OF OTHER SKIN DISEASES**
SALBE ZUR BEHANDLUNG VON VERBRENNUNGEN UND ANDEREN HAUTKRANKHEITEN
POMMADE DESTINEE AU TRAITEMENT DE BRULURES ET D'AUTRES MALADIES CUTANEES

(30) Priority: 07.05.1997 RO 9700849
(43) Date of publication of application: 05.01.2000
(73) Proprietor: S.C. Farmafass Italia SRL, 87068 Rossano (CS) (IT)
(72) Inventor: Stoica, Felician Titus, Timisoara (RO)
(74) Representative: Petra, Elke, Dipl.-Ing.
(86) International application number: RO9800003
(87) International publication number: WO9850055

(56) References cited:
- DATABASE WPI Section Ch, Week 9525 Derwent Publications Ltd., London, GB; Class B04, AN 95-191942 XP002072914 & RO 108 643 B (BRATU I) cited in the application
- DATABASE WPI Section Ch, Week 9813 Derwent Publications Ltd., London, GB; Class B04, AN 98-143665 XP002072915 & RU 2 085 204 C (MAKEEV B A)
- DATABASE WPI Section Ch, Week 9718 Derwent Publications Ltd., London, GB; Class B04, AN 97-200618 XP002072916 & RU 2 066 194 C (BOBROV-EGOROV N N)
- DATABASE WPI Section Ch, Week 9705 Derwent Publications Ltd., London, GB; Class B04, AN 97-049981 XP002072974 & RU 2 027 440 C (MAKEEV B A)

## Description

### Technical Field.

The present invention refers to an ointment used for the treatment of both superficial and profound burns of the skin, as well as for the treatment of other skin diseases

### Backgroung Art.

There are known various pharmaceuticals used for the cure of burns by the control of the microbial development at the level of the lesions provoked by them. They are based on synthetic chemical substances as active components, which are used alone or in combinations. These substances are usually synthetized with difficulty and as a consequence are not so available.

Ointments associating active components having anti-inflammatory, cicatricant and epithelising action are known. Thus, the Romanian patent RO 92445 discloses an ointment for the treatment of skin burns constituted of subnitric bismuth, ichthyole, zinc oxide, camphor, oleum Jecoris, vitamin A acetate, vitamin F, ascorbic acid, hydrocortisone acetate, neomycin sulphate, lanoline and vaseline.

Another known ointment, disclosed in the Romanian patent RO 108.643, consists of white beeswax, colophony, incense, powder of bismuth subgallate and edible sunflower oil, in a weight ratio of 5...20: 2...15: 0.1...0.3 : 0.5...2: 70...90. This ointment, like other known ointments, has a not enough rapid action for burns curing, especially for those which are profound.

The Russian patent RU 2.085.204 refers to an oil-based balsam for wounds healing, comprising more than 0.3% camphor, more than 0.3% an oil concentrate (50% mint oil + 50% other plants oils) and an oil extract of various medicinal herbs as balance. Another Russian patent, RU 2.066.194, refers to a balsam used in different fields of medicine, including dermatology, comprising a mixture of medicinal herbs, an aromatising plant extract and a vegetable oil. The balsams of both patents are based only on plants extracts as active components; they don't refer to rapid healing of skin burns.

### Disclosure of the Invention.

The ointment disclosed in the present invention is based on natural products used as active components, part of these components having known effects on the human body. The association of all these components of the ointment according to the invention allows not only to enlarge the range of pharmaceutical products of this sort, but also to eliminate the synthetic chemical substances and to achieve complex and superior cure effects.

The ointment, according to the invention, consists of 45....67 % edible sunflower oil, 15....37 % edible olive oil, 1....2 % extract from the plant *Calendulae officinalis*, 0.8....1.8 % incense, 2.5.... 8 % colophony, 6.5....11.5 % white beeswax, 0.7.... 1.8 % bismuth subgallate and 0,7.... 1.8 % camphor, the percentages being expressed by weight.

All the components of the ointment according to the invention must be pure, their properties must be those imposed to substances used in pharmaceutical products. Thus, for example, the sunflower oil and olive oil must be freshly prepared and, before their use for the ointment preparation, they are subjected to analyses. These oils are stored in closed vessels, cooled and in the dark. They also must have no water traces, so as to avoid the turbidity of solutions. These oils can be subjected to filtration in the absence of humidity, preferably heated for fluidization, before their use. The preservation of these oils is limited in time. The beeswax is also purified before use.

The active components of the ointment, according to the invention, are reciprocally activating one another and thus they have a synergistic effect in the treatment of some skin diseases or of skin burns, even burns of IIIrd or IVth degree. This ointment, compared to similar, known products, shows the following advantages:
- rapid action on the skin, producing its healing in a short period of time, for instance the tegument epithelization in case of IIIrd degree burns in maximum 14 days,
- a large bacteriostatic and fungicidal spectrum due to the big number of components and to the proportion between them; the ointment has also a limited bactericidal spectrum;
- it does not contain synthetic substances, hardly available.

In the following, examples of producing the ointment according to the invention are presented, in order to explain the invention more detailed.

### Example 1.

In a stainless metal vessel, equipped with mechanical stirrer, thermometer, heating - cooling jacket and with a lid equipped with a ventilation device and a manometer, 415 ml of edible sunflower oil (having a density of 0.920 kg/l) and 675 ml edible olive oil (having a density of 0.9118 kg/l) are fed and then the mixture is heated under stirring up to a temperature of 145°C.

Separately, a mixture of 20 g extract from the plant *Calendulae officinalis* in 400 ml sunflower oil (representing 5 % by weight plant extract in oil) is prepared and then poured, with continuous stirring, into the vessel. The mixture thus obtained is stirred for a period of 5...10 minutes, keeping the temperature at the value of 145°C. Continuing the stirring, 30 g incense are fed into the vessel, they are solved in the existing mixture during 10 minutes, then 100 g colophony are introduced, stirring continues for another period of 8....10 minutes for homogenization, after that 116 g of white beeswax are introduced and the mixture is stirred again until its complete homogenization, which is checked after stopping the stirring and raising up the lid. If it is considered necessary, after closing the vessel again, stirring is continued at the same temperature. After that, 25 g bismuth subgallate (bismuth basic gallate) are fed under stirring and the mixture is cooled to a temperature of 40°C, then 11.7 g camphor are added also under stirring during a period of 8....10 minutes. At the bottom of the vessel where this composition was prepared there is a pipe manufactured from the same stainless metal as the vessel, equipped with a tap, through which the final fluid composition flows into a dosage device, equipped with a codling system, and the ointment thus obtained in this device is canned in a manner known per se in tubes made of pharmaceutically approved materials.

The extract from the plant *Calendulae officinalis* is prepared as disclosed in the following. The plant is harvested only in the morning, when the flower cup is not opened. The stalk, the leaves and the flowers of the plant are cut to a dimension of 1....2 cm, then are mixed together and introduced in a vessel where ethyl alcohol is added. They are let to stay for 10 minutes, then . the ethyl alcohol is drawn out from the vessel, the vessel is closed and maintained closed for a period of 24 hours. The edible sunflower oil is added, the mixture is heated for a period of 24 hours to a temperature of about 60....70°C, stirring the mixture from time to time. Finally it is filtered under pressure and a plant extract of 5% by weight in oil is obtained. This process for the preparation of the plant extract is based on a maceration, but a double maceration can be performed using known procedures in this field. The extract is preserved in well closed vessels, in the dark and at a temperature of 6...8°C.

### Example 2.

An ointment according to the invention is prepared using the procedure discosed in example 1, but different amounts of components, namely: 615 ml edible sunflower oil, 275 ml edible olive oil. a separately, previously prepared mixture of 27.4 g extract of *Calendulae officinalis* and 595 ml sunflower oil (the mixture having 5 % by weight plant extract), 15 g incense, 50 g colophony, 166 g white beeswax and 30 g bismuth subgallate. The mixing of these components and the composition homogenization are performed at the temperature of 185°C. Then, the composition is cooled at a temperature of 45°C and 11.7 g camphor are added to it. Finally, the discharge of the fluid composition from the vessel, its cooling and the canning of the ointment are performed in the same manner as described in example 1.

This composition contains more plant extract and more beeswax, and less incense and colophony compared to the composition prepared in example 1.

### Example 3.

An ointment is prepared using the procedure disclosed in example 1, but introducing in the vessel 217 ml edible sunflower oil, 546 ml edible olive oil, a previously prepared mixture of 33.2 g extract of *Calendulae officinalis* in 685 ml edible sunflower oil, 13.3 g incense, 133 g colophony, 111 g white beeswax and 11.6 g bismuth subgallate. The mixing of the components and the homogenization of the composition thus obtained are performed at a temperature of 200°C, then the mixture is cooled to a temperature of 60°C and 30 g camphor are added to it. Finally, the same steps as those described in example 1 are performed.

Compared to the ointments prepared in examples 1 and 2, this composition has bigger amounts of plant extract, colophony and camphor and a smaller amount of beeswax.

### Example 4.

An ointment is prepared using the procedure disclosed in example 1, but introducing in the vessel 740 ml edible sunflower oil, 400 ml edible olive oil, a previously prepared mixture of 16.6 g extract of *Calendulae officinalis* and 343 ml sunflower oil (having 5 % by weight plant extract), 25 g incense, 42 g colophony, 190 g white beeswax and 11.6 g bismuth subgallate. The mixing of components and the homogenization of the composition thus obtained are performed at a temperature of 160°C, then the mixture is cooled to a temperature of 45°C and 13.3 g camphor are added to it. Finally, the same steps as those described in example 1 are performed.

The ointment according to the present invention is used especially for healing burns. In the case of burns of IIIrd degree, independent on their position on the human body, a layer of the ointment having a thickness of 2...3 mm is applied on a sterile dressing and then the dressing is applied on the affected zone. In the first 4...6 days, the dressing is changed after every 24 hours in the following manner: the dressing is removed, on the affected place camomile tea is sprayed, the plasmorrhage is removed with very much easiness compared to the treatments using other ointments, another spraying with camomile tea is perfomed, the zone is dried by wadding it with a sterile dressing and another dressing having on it a layer of ointment is applied. Starting with the 7-th day and until the 14-th day of treatment, or even sooner, depending on the seriousness of the disease and on the affected surface, the dressing is changed after every 48 hours. After the epithelium recovery, the treatment is continued by applying a very thin layer of ointment (by wadding) on the cured zone, in order to avoid the tegument fissures.

In case of burns of IVth degree, the plasmorrhage removing must be performed with very much attention so as to avoid cheloid formation.

The ointment, according to the invention, can be used with very good results in shank ulcer. In this situation, the thickness of the ointment layer on the dressing is of 10 mm, in order to be possible to have a good absorbent bed for the plasmorrhage which was formed. The treatment duration is of 4....8 weeks, depending on the surface and the deepness of the wound.

Another use of the ointment according to the invention is in gynecology, for the treatment of cervicitis. In this case, a small sterile dressing, well imbued with ointment is used, being applied in the affected zone. It is changed after every 24 hours and the place is cleaned with plenty of camomile tea. The treatment duration is of 2....3 weeks depending on the seriousness of the cervicitis.

## Claims

1. Ointment for the treatment of burns and of other skin diseases **characterized in that** it consists of 45....67 % edible sunflower oil, 15....37 % edible olive oil, 1....2% extract in ethanol from the plant *Calendulae officinalis*, 0.8....1.8 % incense, 2.5....8 % colophony, 6.5....11.5 white beeswax, 0.7....1.8 % bismuth subgallate and 0.7....1.8 % camphor, the percentages being expressed by weight.

2. Ointment for the treatment of burns and of other skin diseases **characterized in that** it consists of 749.8 g edible sunflower oil, 615.46 g edible olive oil, 20 g extract in ethanol from the plant *Calendulae officinalis*, 30 g incense, 100 g colophony, 116 g white beeswax, 25 g bismuth subgallate and 11.7 g camphor.

3. Ointment for the treatment of burns and of other skin diseases **characterized in that** it consists of 1113.2 g edible sunflower oil, 250.75 g edible olive oil, 27.4 g extract in ethanol from the plant *Calendulae officinalis*, 15 g incense, 50 g colophony, 166 g white beeswax, 30 g bismuth subgallate and 11.7 g camphor.

4. Ointment for the treatment of burns and of other skin diseases **characterized in that** it consists of 829.84 g edible sunflower oil, 497.84 g edible olive oil, 33.2 g extract in ethanol from the plant *Calendulae officinalis,* 13.3 g incense, 133 g colophony, 111 g white beeswax, 11.6 g bismuth subgallate and 30 g camphor.

5. Ointment for the treatment of burns and of other skin diseases **characterized in that** it consists of 996.36 g edible sunflower oil, 364.72 g edible olive oil, 16.6 g extract in ethanol from the plant *Calendulae officinalis,* 25 g incense, 42 g colophony, 190 g white beeswax, 11.6 g bismuth subgallate and 13.3 g camphor.

## Patentansprüche

1. Salbe zur Behandlung von Verbrennungen und anderen Hautkrankheiten, **dadurch gekennzeichnet, daß** sie aus 45...67 % eßbarem Sonnenblumenöl, 15...37 % eßbarem Olivenöl, 1...2 % ethanolischem Extrakt aus der Pflanze Calendulae officinalis, 0,8... 1,8 % Weihrauch, 2,5...8 % Kolophonium, 6,5...11,5 % weißem Bienenwachs, 0,7...1,8 % subgallischem Wismut (basischem Wismutgallat) und , 0,7...1,8 % Campher besteht, wobei die Anteile in Gewicht angegeben sind.

2. Salbe zur Behandlung von Verbrennungen und anderen Hautkrankheiten, **dadurch gekennzeichnet, daß** sie aus 749,8 g eßbarem Sonnenblumenöl, 615,46 g eßbarem Olivenöl, 20 g ethanolischem Extrakt aus der Pflanze Calendulae officinalis, 30 g Weihrauch, 100 g Kolophonium, 116 g weißem Bienenwachs, 25 g subgallischem Wismut und 11,7 g Campher besteht.

3. Salbe zur Behandlung von Verbrennungen und anderen Hautkrankheiten, **dadurch gekennzeichnet, daß** sie aus 1113,2 g eßbarem Sonnenblumenöl, 250,75 g eßbarem Olivenöl, 27,4 g ethanolischem Extrakt aus der Pflanze Calendulae officinalis, 15 g Weihrauch, 50 g Kolophonium, 166 g weißem Bienenwachs, 30 g subgallischem Wismut und 11,7 g Campher besteht.

4. Salbe zur Behandlung von Verbrennungen und anderen Hautkrankheiten, **dadurch gekennzeichnet, daß** sie aus 829,84 g eßbarem Sonnenblumenöl, 497,84 g eßbarem Olivenöl, 33,2 g ethanolischem Extrakt aus der Pflanze Calendulae officinalis, 13,3 g Weihrauch, 133 g Kolophonium, 111 g weißem Bienenwachs, 11,6 g subgallischem Wismut und 30 g Campher besteht.

5. Salbe zur Behandlung von Verbrennungen und anderen Hautkrankheiten, **dadurch gekennzeichnet, daß** sie aus 996,36 g eßbarem Sonnenblumenöl, 364,72 g eßbarem Olivenöl, 16,6 g ethanolischem Extrakt aus der Pflanze Calendulae officinalis, 25 g Weihrauch, 42 g Kolophonium, 190 g weißem Bienenwachs, 11,6 g subgallischem Wismut und 13,3 g Campher besteht.

## Revendications

1. Pommade destinée au traitment des brûlures et d'autres maladies cutanées **caracterisé en ce qu'**elle est constitué de 45...67% huile de tournesol alimentaire, 15...37% huile d'olives alimentaire, 1...2% extrait en éthanol de Calendulae officinalis, 0,8...1,8% encens, 2,5...8% colophane, 6,5...11,5% cire blanche d'abeilles, 0,7... 1,8% bismuth subgallate et 0,7...1,8% camphre, les pourcentages etant exprimé en poids.

2. Pommade destinée au traitement des brûlures et d'autres maladies cutanées **caracterisé en ce qu'**elle est constitué de 749,8 g huile de tournesol alimentaire, 615,46 g huile d'olives alimentaire, 20 g extrait en éthanol de Calendulae officinalis, 30 g encens, 100 g colophane, 116 g cire blanche d'abeilles, 25 g bismuth subgallate et 11,7 g camphre.

3. Pommade destinée au traitement des brûlures et d'autres maladies cutanées **caracterisé en ce qu'**elle est constitué de 1113,2 g huile de tournesol alimentaire, 250,75 g huile d'olives alimentaire, 27,4 g extrait en éthanol de Calendulae officinalis, 15 g encens, 50 g colophane, 166 g cire blanche d'abeilles, 30 g bismuth subgallate et 11,7 g camphre.

4. Pommade destinée au traitement des brûlures et d'autres maladies cutanées **caracterisé en ce qu'**elle est constitué de 829,84 g huile de tournesol alimentaire, 497,84 g huile d'olives alimentairel, 33,2 g extrait en éthanol de Calendulae officinalis, 13,3 g encens, 133 g colophane, 111g cire blanche d'abeilles, 11,6 g bismuth subgallate et 30 g camphre.

5. Pommade destinée au traitement des brûlures et d'autres maladies cutanées **caracterisé en ce qu'**elle est constitué de 996,36 g huile de tournesol alimentaire, 364,72 g huile d'olives alimentaire, 16,6 g extrait en éthanol de Calendulae officinalis, 25 g encens, 42 g colophane, 190 g cire blanche d'abeilles, 11,6 g bismuth subgallate et 13,3 g camphre.
